# EUROPÄISCHE PATENTSCHRIFT

(11) **EP 0 670 904 B1**
(45) Veröffentlichungstag und Bekanntmachung des Hinweises auf die Patenterteilung: **19.03.2003**
(21) Anmeldenummer: 94900042.6
(22) Anmeldetag: 10.11.1993
(51) Int. Cl.: C12N 15/85

(54) **VEKTOR ZUR EXPRESSION VON THERAPEUTISCH RELEVANTEN GENEN**
VECTOR FOR THE EXPRESSION OF THERAPY-RELEVANT GENES
VECTEUR D'EXPRESSION DE GENES UTILES EN THERAPIE

(30) Priorität: 12.11.1992 DE 4238778
(43) Veröffentlichungstag der Anmeldung: 13.09.1995
(73) Patentinhaber: MAX-DELBRÜCK-CENTRUM FÜR MOLEKULARE MEDIZIN, 13125 Berlin (DE)
(72) Erfinder: STEIN, Ulrike, D-13125 Berlin (DE); WALTHER, Wolfgang, D-13125 Berlin (DE)
(74) Vertreter: Baumbach, Friedrich, Dr.
(86) Internationale Anmeldenummer: DE9301086
(87) Internationale Veröffentlichungsnummer: WO94011522

(56) Entgegenhaltungen:
- WO-A-89/12109
- BIOCHEMICAL AND BIOPHYSICAL RESEARCH COMMUNICATIONS Bd. 165, Nr. 3 , 29. Dezember 1989 , DULUTH, MINNESOTA US Seiten 1415 - 1421 K. KOHNO ET AL. 'Th direct activation of human multidrug resistance gene (MDR1) by anticancer agents'
- JOURNAL OF BIOLOGICAL CHEMISTRY. (MICROFILMS) Bd. 265, Nr. 32 , 15. November 1990 , BALTIMORE, MD US Seiten 19690 - 19696 K. KOHNO ET AL. 'Tissue-specific enhancer of the human multidrug-resistance (MDR1) gene' in der Anmeldung erwähnt
- PROCEEDINGS OF THE NATIONAL ACADEMY OF SCIENCES OF USA. Bd. 86 , 1989 , WASHINGTON US Seiten 3519 - 3523 P. A. HANTZOPOULOS ET AL. 'Improved gene expression upon transfer of the adenosine demainase minigene outside the transcriptional unit of a retroviral vector' in der Anmeldung erwähnt

## Beschreibung

Die Erfindung betrifft einen Vektor und seine Verwendung zur Expression von therapeutisch relevanten Genen in Säugerzellen. Anwendungsgebiete der Erfindung sind die pharmazeutische Industrie und die Medizin.

Bei der Behandlung von Krebspatienten mit Zytostatika treten oftmals Resistenzerscheinungen auf (Igor. B. Roninson. Molecular and cellular biology of multidrug resistance in tumor cells. Plenum Press, New York and London, 1991), die dem Therapieerfolg hinderlich sind. Es wurden deshalb viele verschiedene Chemotherapie-Regime mit Variationen der Zytostatika-Applikation und deren Dosierung entwickelt. Darüberhinaus ist eine Verbesserung der Chemotherapie durch Kombinationen von Zytostatika mit anderen Substanzklassen, wie z.B. Cytokinen, monoklonalen Antikörpern etc. versucht worden. Ein weiterer Weg für eine effektivere Gestaltung einer Tumorchemotherapie besteht im Einsatz von Antikörper-gekoppelten Zytostatika, um damit einen Transport des Zytostatikums zur Krebszelle und ein Wirksamwerden in dieser Zelle zu erreichen (H. M. Pinedo et al. Cancer chemotherapy and biological response modifiers. Annual 12. Elsevier, Amsterdam-New York-Oxford, 1991). Trotz einiger Teilerfolge konnte jedoch ein durchgreifender klinischer Erfolg noch mit keiner dieser Methoden erreicht werden.

Die Erfindung hat das Ziel, die Krebstherapie effektiver und somit erfolgreicher zu gestalten. Die Aufgabe besteht insbesondere darin, ein eingesetztes Therapeutikum (Zytostatikum) mit einem zweiten Therapeutikum (antitumoral wirkendes Protein), das erst in den Krebszellen entsteht und somit unmittelbar auf die Krebszelle wirkt, zu kombinieren.

Das Ziel der Erfindung wird mit der Verwendung eines Expressionsvektors gemäß der Ansprüche 1-3 erreicht im Anspruch 4 wird der erfindungsgemäße Vektor näher gekennzeichnet.

Die Erfindung nutzt die Eigenschaft des mdr1-Gens aus, daß sein Genprodukt P-Glycoprotein (verantwortlich für den Resistenztyp der Arzneimittel-Vielfachresistenz) durch Zytostatika wie Vincristin und Adriamycin induzierbar ist. Diese Zytostatika-induzierbare Genexpression wird über bestimmte Promoter- und Enhancer -Elemente des mdr1-Gens vermittelt. Überraschenderweise wurde gefunden, daß diese Promoter- und Enhancer-Elemente auch in der Lage sind, andere Gene - z.B. Gene von therapeutischem Interesse wie Cytokin-Gene oder Gene für monoklonale Antikörper - zu exprimieren. Der erfindungsgemäße Vektor gemäß Anspruch 4 ermöglicht damit eine Zytostatika-induzierte Fremdgenexpression in der Körperzelle und somit eine Kombination von Chemo- und Immuntherapie "vor Ort".

Als Grundgerüst für die erfindungsgemäßenVektoren sind alle Konstrukte verwendbar, die für eine Expression in Säugerzellen geeignet sind, wobei neben z.B. Adenovirus-Vektoren vor allem retroviralen Expressionsvektoren (N2A, pM3-neo, pM5-neo) der Vorrang gegeben wird. Die Vektorkonstruktionen werden gezielt mit einem tumorspezifischen Targeting unter Verwendung von geeigneten Carriersystemen, wie Immunoliposomen, Retroviruspartikeln etc. in an sich bekannter Weise verbunden, damit eine Transport zu den Zielzellen im Patienten ermöglicht wird. Dort werden sie in die Zellen inkorporiert und können dann ihre therapeutische Wirkung entfalten, d.h. auf die danach einsetzende Chemotherapie hin werden in den Tumorzellen über die induzierbaren Elemente antitumorale Substanzen in die Zellen und die Tumorumgebung freigesetzt.

Mit Hilfe der Erfindung kann die Chemotherapie also mit den antitumoralen Effekten der induzierten Proteine auf neue Weise kombiniert werden.

Die erfindungsgemäßen Konstrukte agieren intrazellulär; mit einer Chemotherapie wird durch bestimmte Zytostatika über die Promotoren/Enhancer die Expression des Fremdgens induziert, das dann direkt "vor Ort" seine antitumorale Wirkung entfalten kann. Auf diesem Wege wirkt auf die Zielzelle (Tumorzelle) gleichzeitig das Zytostatikum und das Fremdgenprodukt, das darüberhinaus auch noch einen systemischen Effekt ausüben kann. Für die Cytokine z.B. bedeutet das, daß sie neben ihrer unmittelbaren zytotoxischen/zytostatischen Wirkung an der Zielzelle auch Effekte auf die Immunabwehr des Organismus haben, wie z.B. die Aktivierung von T-Lymphozyten, Makrophagen etc. Sie wirken unterstützend bei einer Tumortherapie.

Die Erfindung soll nachstehend durch Ausführungsbeispiele näher erläutert werden.

### AUSFÜHRUNGSBEISPIEL 1

### Konstruktion eines Vektors CVS-SW1 zur Zytostatika-induzierbaren Expression eines therapeutisch relevanten Gens

### Polymerase-Kettenreaktion (PCR)

Es wird die hochmolekulare humane DNA aus in vitro-Zellinien oder Tumormaterial in an sich bekannter Weise isoliert. Mit der PCR (1 min 94°C; 1 min 45°C; 2 min 72°C; 30 Zyklen) werden dann die unter den Ansprüchen 2a) bis d) bzw. 3a) und b) genannten Promoter-Sequenzen des mdr1-Gens bzw. die unter 4. genannten Enhancer-Sequenzen des mdr1-Gens (Kimitoshi Kohno et al. J. Biol. Chem. 265: 19690-1969b, 1990) amplifiziert.

Im Falle dieses Konstruktionsbeispiels werden zur PCR der mdr1-Promoter-Sequenzen Primer eingesetzt (17 Basen der 5'- bzw. 3'-mdr1-Promotersequenz + Basen der jeweiligen Restriktionsendonukleasen + Schutzgruppe TAC), an deren 5'-Ende sich spezifische Sequenzen zur Apa I-Klonierung (GGGCCC) und am 3'-Ende Sequenzen zur Sma I (CCCGGG)- und Bgl II (AGATCT)-Klonierung befinden.

Die Amplifikation der unter Anspruch 4. genannten Enhancer-Sequenz des mdr1-Gens erfolgt in diesem Beispiel mit 5'-Sst II (CCGCCG)- und Sma I (CCCGGG)- Primern und 3'-Mlu I (ACGCG)-Primern (wiederum 17 Basen der 5'- bzw. 3'-mdr1-Enhancer-Sequenz + Basen der jeweiligen Restriktionsendonukleasen + Schutzgruppe TAC).

Die erhaltenen Amplifikate der mdrl-Promotcr- bzw. Enhancer-Sequenzen werden dann mit Apa I/Bgl II bzw. mit Sst II/Mlu I gespalten, gereinigt und stehen somit zur Klonierung in den Vektor N2A zur Verfügung.

### Klonierung

Der retrovirale "double copy"-Vektor N2A (Petros A. Hantzopoulos et al. Proc. Natl. Acad. Sci. USA 86: 3519-3523, 1989) mit einer Größe von 9.678 kb wird zunächst Apa I/Bgl IIgespalten und mit Amplifikaten einer mdr1-Promoter-Sequenzausa)bisd)des Anspruches 2 ligiert. Dieses Zwischenkonstrukt I wird in geeignete Bakterien transformiert und über die Methode der Plasmidamplifikation in an sich bekannter Weise aus 500 ml einer Bakteriensuspension (OD₆₀₀=0.4) isoliert. Die Reinigung der Plasmid-DNA erfolgt dann mit bekannten MethodenübereineCsCI-Gradienten-Zentrifugation. Durch erneute Restriktase-Spaltung mit Apa I und Bgl II wird dann aus der gewonnenen Plasmid-DNA das entsprechende Promoter-Fragment in der Agarose-Gelelektrophorese nachgewiesen und die Klonierung somit überprüft.

Dieses Zwischenkonstrukt I (Vektor N2A mit einer Zytostatika-induzierbaren mdr1-Promoter-Sequenz) wird nun mit den Restriktasen Sst II und Mlu I gespalten, um die mdr1-Enhancer-Sequenz (5'-Sst II; 3'-Mlu I) mit dem Zwischenkonstrukt I zu ligieren. Das nun erhaltene Zwischenkonstrukt II wird wiederum in Bakterien transformiert und im Maßstab einer 500 ml-Bakterienkultur vermehrt und isoliert. Die ebenfalls CsCl-Gradienten-gereinigte Plasmid-DNA wird zur Kontrolle der Klonierung mit Sst II/Mlu I gespalten. Die Größen der Spaltfragmente werden wiederum in der Agarose-Gelelektrophorese überprüft.

Damit steht nun ein Kassetten-Vektorsystem CVS-SW1 zur Klonierung (z.B. im Sma I-Ort oder im Sna BI-Ort) von therapeutisch interessanten Fremdgenen wie z.B. von Cytokinen, monoklonalen Antikörpern oder Antionkogenen zu deren Zytostatika-induzierbarer Expression "vor Ort" als gentherapeutisches Prinzip zur Verfügung (Abb. 1).

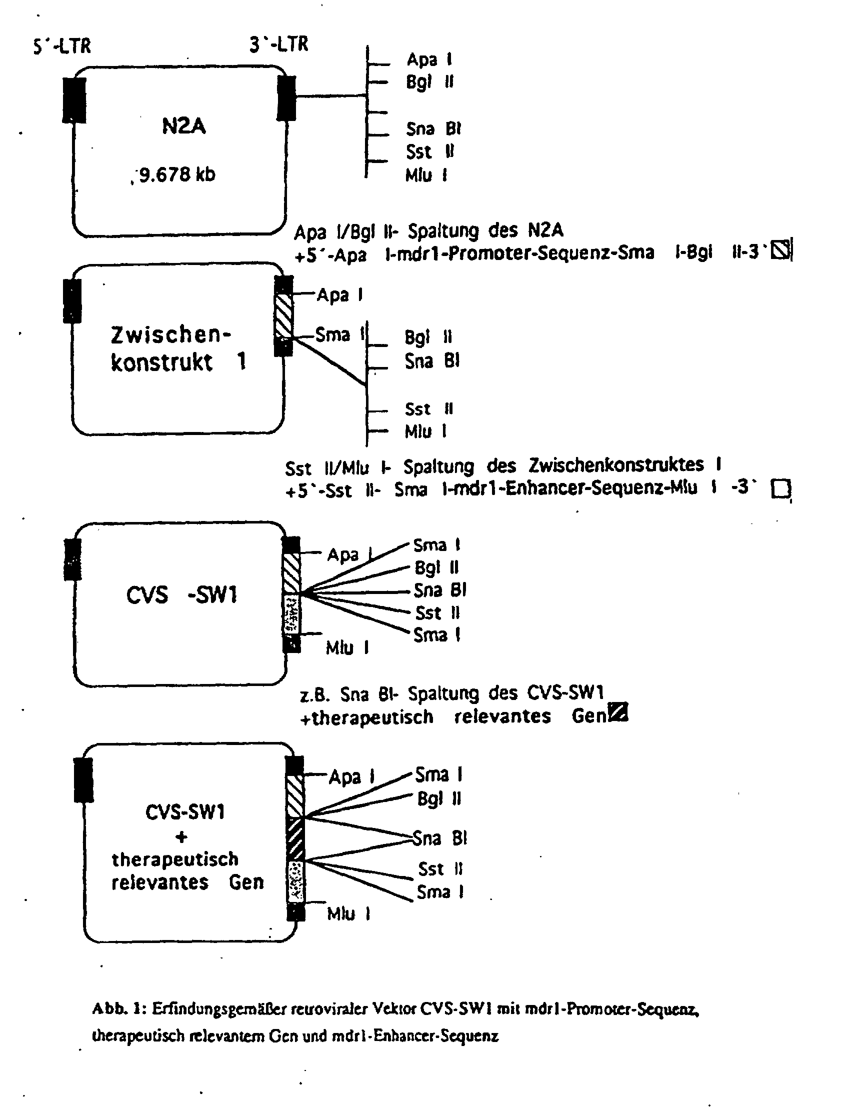

### Induktion eines Fremdgens im Kassetten-Vektorsystem CVS-SW1

Die Induzierbarkeit eines Fremdgens im Kassetten-Vektorsystem CVS-SW1 wird am Beispiel des CAT-Gens (Chloramphenikolazetyltransferase) nach Transfektion des Konstruktes CVS-SW1 mit dem CAT-Gen in COS-Zellen mittels CAT-ELISA (Enzymimmunoassay zur quantitativen Bestimmung von Chloramphenikolazetyltransferase in transfizierten, eukaryontischen Zellen) überprüft.

Die Testung der Induzierbarkeit eines therapeutisch relevanten Gens erfolgt über Expressionsanalysen auf RNA- und Proteinebene und über die Testung der biologischen Aktivität entsprechend der Eigenschaften und Funktion des Fremdgenproduktes.

### Liposomale Verkapselung und Applikation der Konstrukte

Die Verkapselung der Zytostatika-induzierbaren Kassetten-Vektorsysteme (z.B. CVS-SW1) mit einem therapeutisch relevanten Gen, das gewebe- bzw. tumorspezifische Targeting durch die Liposomen und die Einschleusung in in vitro-Zellkulturen sowie in in vivo-Systeme (nude-Maus) erfolgt in an sich bekannter Weise nach NICOLAU (Claude Nicolau. Amelia Cudd. Critical reviews in therapeutic drug carrier systems 6: 239-271, CRC Press, Inc, 1989) und HUG (Peter Hug, Richard G. Steight. Biochim. Biophys. Acta 1097: 1-17, 1991).

Eine perspektivische Anwendung am Krebspatienten orientiert sich an verschiedenen schon durchgeführten Studien (A. Dusty Miller. Nature 357: 455-460, 1991; Kenneth W. Culver et al. Science 256: 1550-1552, 1992; Zvi Ram. Cancer Res. 53: 83-88, 1993).

### Transduktion in eine Verpackungszellinie

Die Verpackung der Zytostatika-induzierbaren Kassetten-Vektorsysteme mit einem therapeutisch relevanten Gen erfolgt über die Transduktion der Konstrukte in eine Verpackungszellinie. Eine dafür besonders geeignete Linie ist die Verpackungslinie PA 317.

### AUSFÜHRUNGSBEISPIEL 2

### Konstruktion eines Vektors CVS-SW2 zur Zytostatika-induzierbaren Expression eines therapeutisch relevanten Gens

In ähnlicher Weise, jedoch mit entsprechend veränderten Klonierungsorten erfolgt die Klonierung des Kassetten-Vektorsystems CVS-SW2 mit der Reihenfolge: 1. mdr1-Enhancer-Sequenz, 2. mdr1-Promoter-Sequenz und 3. dem therapeutisch interessanten Gen.

### AUSFÜHRUNGSBEISPIELE 3 UND 4

### Konstruktion der Vektoren CVS-SW3 und CVS-SW4 zur Zytostatika-induzierbaren Expression von therapeutisch relevanten Genen

In ähnlicher Weise wie in den Ausführungsbeispielen 1 und 2, jedoch mit entsprechend veränderten Klonierungsorten und einer vorhergehenden Klonierung im Vektor Bluescript M13+ erfolgt die Klonierung der Kassetten-Vektorsysteme CVS-SW3 und 4. Es handelt sich hierbei um die mutierten mdr1-Promoter-Sequenzen des Anspruches 3a) und b). Für die Klonierung des therapeutisch relevanten Gens in die Vektoren CVS-SW3 bzw. CVS-SW4 stehen außer denen in den Ausführungsbeispielen 3 bzw. 4 genannten Spaltorten ebenso die Orte Bam HI, Spe I, Xba I, Eag I und Not 1 zur Verfügung (Abb. 2).

In analoger Weise sind auch andere eukaryontische Expressionsvektoren zu einer Zytostatika-induzierbaren Fremdgenexpression nutzbar. Die weiteren Schritte zur Induktion eines Fremdgens in den Kassetten-Vektorsystem CVS-SW2, 3 und 4, zur liposomalen Verkapselung des Konstruktes bzw. seiner Transduktion in eine Verpackungszellinie bis hin zur Applikation schließen sich, wie im Ausführungsbeispiel 1 genannt, an.

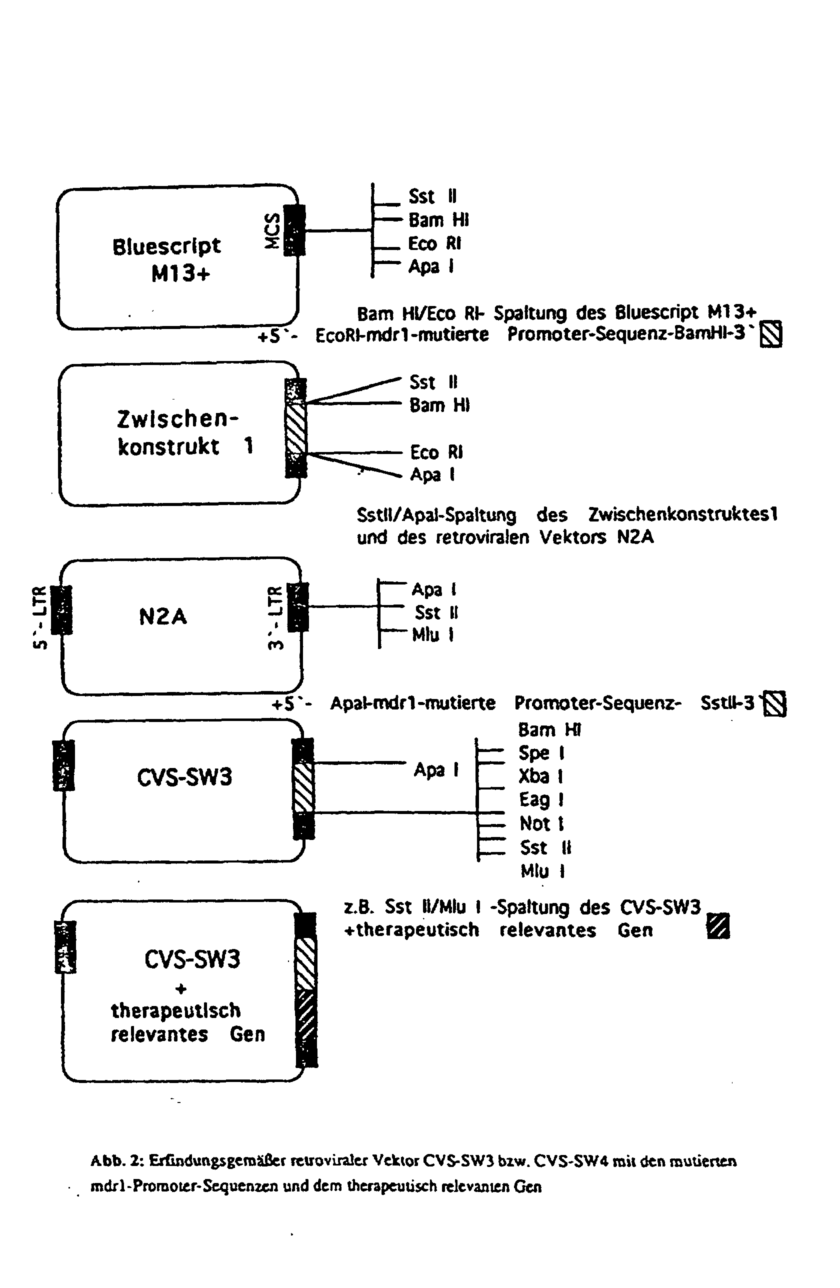

## Patentansprüche

1. Verwendung eines Expressionsvektors, der aus
a) einem Zystostatika-induzierbaren mdr1-Promotor bzw. einer definierten Sequenz davon
b) einem Gen, das für ein therapeutisch relevantes Protein kodiert, und
c) ggf. vor oder nach dem Gen angeordneten mdr1-Enhancer-Elementen
besteht, zur Herstellung eine Therapeutikums, **gekennzeichnet durch** eine Verpackung dieses Retrovirus- oder Adenovirus-abgeleiteten Vektors in Carrier-Systemen, bevorzugt Immunoliposomen oder Retroviruspartikeln.

2. Verwendung des Expressionvektors nach Anspruch 1 zur Herstellung amphotroper Retroviruspartikel für einen Retrovirus-vermittelten Gentransfer, **gekennzeichnet durch** Transduktion und Integration in einer Verpackungszellinie.

3. Verwendung des Expressionvektors nach Anspruch 1 und 2, **dadurch gekennzeichnet, dass** als Verpackungslinie die Linie PA 317 eingesetzt wird.

4. Spezifisches Konstrukt, bestehend aus
a) folgenden mutierten Sequenzen des Zytostatika-induzierbaren mdr-1-Promoter
Sequenz: Transition ö103 T->C Sequenz: Transversion +137 G->T
b) ein therapeutisch relevantes Protein codierendes Gen
c) gegebenenfalls vor oder nach dem Gen angeordnete mdr-1-Enhaucer-Elemente

## Claims

1. Use of an expression vector comprising
a) a cytostatic-inducible mdr1 promotor or a defined sequence thereof, as the case may be,
b) a gene coding for a therapeutically relevant protein and
c) if need be, mdr1 enhancer elements arranged in front or behind the gene,
for the production of a therapeutic agent, wherein there exists a packaging of this retrovirus or adenovirus-derived vector in carrier systems, preferably immunoliposomes or retrovirus particles.

2. Use of an expression vector according to Claim 1 for the production of amphotropic retrovirus particles for a retrovirus-mediated gene transfer, wherein there exists transduction and integration in a packaging cell line.

3. Use of the expression vector according to Claims 1 and 2, wherein the line PA 317 is used as a packaging line.

4. Specific construction, comprising
a) the following mutated sequences of the cytostatic-inducible mdr1 promotor
sequence: transition +103 T->C sequence: transversion +137 G->T
b) a therapeutically relevant protein coding gene
c) if need be, mdr1 enhancer elements arranged in front or behind the gene.

## Revendications

1. Emploi d'un vecteur d'expression qui se compose
a) d'un promoteur mdr1 inductible de cystostatiques resp. d'une séquence définie de celui-ci
b) d'un gène codant pour une protéine d'importance thérapeutique et
c) le cas échéant d'éléments renforceurs de mdr1 placés avant ou après le gène
pour fabriquer un agent thérapeutique, se caractérisant par un emballage de ce vecteur dérivé du rétrovirus ou de l'adénovirus dans des systèmes porteurs, de préférence des immunoliposomes ou des particules de rétrovirus.

2. Emploi du vecteur d'expression selon la revendication 1 pour fabriquer des particules de rétrovirus amphotrophiques pour un transfert génétique établi par un rétrovirus, se caractérisant par la transduction et l'intégration dans une ligne cellulaire d'emballage.

3. Emploi du vecteur d'expression selon la revendication 1 et 2, se caractérisant par le fait que la ligne PA 317 sera employée en tant que ligne d'emballage.

4. Construction spécifique se composant de
a) séquences mutées du promoteur mdr1 inductible de cystostatiques suivantes:
→ Séquence: transition + 103 T -> C
→ Séquence: transversion + 137 G-> T
b) un gène codeur de protéine d'importance thérapeutique
c) le cas échéant d'éléments renforceurs de mdr1 placés avant ou après le gène


### SEQUENZPROTOKOLL

### (1) ALLGEMEINE ANGABEN:

(i) ANMELDER:
   (A) NAME: MAX-DELBRUECK CENTRUM FUER MOLEKULARE MEDIZIN
   (B) STRASSE: Robert-Roessle-Strasse 10
   (C) ORT: Berlin
   (E) LAND: Deutschland
   (F) POSTLEITZAHL: D-13125
   (G) TELEFON: 030/9406-3432
   (H) TELEFAX: 030/9406-2780
(ii) BEZEICHNUNG DER ERFINDUNG: Vektor zur Expression von therapeutisch relevanten Genen
(iii) ANZAHL DER SEQUENZEN: 7
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30B (EPA)
(vi) DATEN DER URANMELDUNG:
   (A) ANMELDENUMMER: DE P 42 38 778.7
   (B) ANMELDETAG: 12-NOV-1992

### (2) ANGABEN ZU SEQ ID NO: 1:

(i) SEQUENZKENNZEICHEN:
   (A) LÄNGE: 2090 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..2090
   (D) SONSTIGE ANGABEN:/note= "Sequenz gem. Anspruch 2 a)"
(xi) SEQUENZBESCHREIBUNG: SEQ ID NO: 1:
   CAAGGACTGT TGAAAGTAGC AAGAGCTAGT TTGTTTTAGG TCCATCATGT TTTATATTCA   60
   CACTTTCATG TCAGTGGAGC AAAGAAATGG AATACAATAT AATAGAATGG TAGAATCTTA  120
   TTTTTAAAAT CTGTGTTATT CTGATCTTTA ACTTACTTAT ATCTTTGATA GAGATCTTTA 180
   CCTGATGCTC AAGATTGTAG AAATAGTATA ATCAACATAA CAGTATAGCA CTGTATTTAT 240
   ATCCTGCACT GTTTAGGGAG GGTTTAAGGC CATTCAAAAG GATACATAAA ATACAACAAG 300
   ATTACATAAA TGAAAGGTGA GATAAAGCAA CAAAGCAAAA CAAAAGTGAA AACAGAGATC 360
   ATAGGCACAA ATAAGATTAA AAACGCATGT AATGAAGATG AAAGCTTTTA CATTTACCCC 420
   AGATGGACCA CAGGGTTGTT GTTAAGCCTT TAAACAGTGA ACAATGCTGT ACACTTGCAT 480
   ATGCAATTAG AACATGTGGA AAAAATAGTG GCCTGTTAGA AGCCTAATTA ACAATTTGTG 540
   AAAAAAAAAA AAAAAAAAAA AAAAAAGAGG CCGAGCTGTA GCTCACGCCT GTAATCCCTG 600
   CACTTTGGGA GGCCGAGGCG GGCGGATCAC GAGGTCAGGA GATCAAGACC ATCCTGGCTA 660
   ACACAGTGAA ACCCAGTCTC TACGAAAAAT ACAAAAAATT AGCCGGGCGT GGTGGCGGGA 720
   GCCTGTAGTC CCAGCTACCT GGGAGGCTGA GGCAGGAGAA TGGTGTGAAC CCGGGAGGCG 780
   GAGCTTGCAG TGAGCCGAGA TCCTGCCACT GCACTCCAGC CTGGGCGACA AAGCAAGCTC 840
   CGTCTGCAAA AAAGAAAAAA GAAAGAAAAA CAAAAGAAAA CTTCATTGTA TTGTAAGGCC 900
   AAGAACAAAA TATATCAAGA TAAGGAAAAT TTGTAGTCAA GAATAGAAAA AAATTATGGC 960
   TTTGAAGTAT GAGTTATTTA AAGAAAGTGG AAACATCCTC AGACTATGCA GTAAAAAACA 1020
   AAGTGATTTT CTTCTTCTAA ACTTATGCAA TAAACTGATA GGTAATATGT GAAAGTCATA 1080
   GAATGTAGAC TAGAGGATAC AACAAACCTA TTTCCTCTAT GTTCATAAGA AGTAAGAAAA 1140
   GCTCTGATGT GAGTTAGCAT TGCTTTACAA TTTTGAATTG TGCAGATTGC CAGTACTTTT 1200
   CCTCAGTTTG AAGTAAATAG TGGACAGGAA AAAATATTAA ATGTTGGCAG TAAATATGGA 1260
   AGGAAATTAC AACTAATGTA ATATGCTAAA ACATGCTATG TTTATTTTAC TAATTTGAAT 1320
   TAAAATGTAA GAATTTAAAA TGCCCTGGAA AAACACGGGC ATTGATCTGA CGTCTGAAGT 1380
   TTTAAAATAT TACACACTTT GAAATAGCAT TTGTACCTTG AAATACCTGT CTCTATATAT 1440
   TTTTTAAAAC TTCCTTTTTC TTTCATTCCA TTTATCATCA AATAAAGGAT GAACAGATGT 1500
   AACTCAGAAA CTGTCAAGCA TGCTGAAGAA AGACCACTGC AGAAAAATTT CTCCTAGCCT 1560
   TTTCAAAGGT GTTAGGAAGC AGAAAGGTGA TACAGAATTG GAGAGGTCGG AGTTTTTGTA 1620
   TTAACTGTAT TAAATGCGAA TCCCGAGAAA ATTTCCCTTA ACTACGTCCT GTAGTTATAT 1680
   GGATATGAAG ACTTATGTGA ACTTTGAAAG ACGTGTCTAC ATAAGTTGAA ATGTCCCCAA 1740
   TGATTCAGCT GATGCGCGTT TCTCTACTTG CCCTTTCTAG AGAGGTGCAA CGGAAGCCAG 1800
   AACATTCCTC CTGGAAATTC AACCTGTTTC GCAGTTTCTC GAGGAATCAG CATTCAGTCA 1860
   ATCCGGGCCG GGAGCAGTCA TCTGTGGTGA GGCTGATTGG CTGGGCAGGA ACAGCGCCGG 1920
   GGCGTGGGCT GAGCACAGCG CTTCGCTCTC TTTGCCACAG GAAGCCTGAG CTCATTCGAG 1980
   TAGCGGCTCT TCCAAGCTCA AAGAAGCAGA GGCCGCTGTT CGTTTCCTTT AGGTCTTTCC 2040
   ACTAAAGTCG GAGTATCTTC TTCCAAGATT TCACGTCTTG GTGGCCGTTC            2090

### (2) ANGABEN ZU SEQ ID NO: 2:

(i) SEQUENZKENNZEICHEN:
   (A) LÄNGE: 1688 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..1688
   (D) SONSTIGE ANGABEN:/note= "Sequenz gem. Anspruch 2 b)"
(xi) SEQUENZBESCHREIBUNG: SEQ ID NO: 2:

### (2) ANGABEN ZU SEQ ID NO: 3:

(i) SEQUENZKENNZEICHEN:
   (A) LÄNGE: 1318 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..1318
   (D) SONSTIGE ANGABEN:/note= "Sequenz gem. Anspruch 2 c)"
(xi) SEQUENZBESCHREIBUNG: SEQ ID NO: 3:
   GGGAGGCGGA GCTTGCAGTG AGCCGAGATC CTGCCACTGC ACTCCAGCCT GGGCGACAAA   60
   GCAAGCTCCG TCTGCAAAAA AGAAAAAAGA AAGAAAAACA AAAGAAAACT TCATTGTATT  120
   GTAAGGCCAA GAACAAAATA TATCAAGATA AGGAAAATTT GTAGTCAAGA ATAGAAAAAA  180
   ATTATGGCTT TGAAGTATGA GTTATTTAAA GAAAGTGGAA ACATCCTCAG ACTATGCAGT  240
   AAAAAACAAA GTGATTTTCT TCTTCTAAAC TTATGCAATA AACTGATAGG TAATATGTGA  300
   AAGTCATAGA ATGTAGACTA GAGGATACAA CAAACCTATT TCCTCTATGT TCATAAGAAG  360
   TAAGAAAAGC TCTGATGTGA GTTAGCATTG CTTTACAATT TTGAATTGTG CAGATTGCCA  420
   GTACTTTTCC TCAGTTTGAA GTAAATAGTG GACAGGAAAA AATATTAAAT GTTGGCAGTA  480
   AATATGGAAG GAAATTACAA CTAATGTAAT ATGCTAAAAC ATGCTATGTT TATTTTACTA  540
   ATTTGAATTA AAATGTAAGA ATTTAAAATG CCCTGGAAAA ACACGGGCAT TGATCTGACG  600
   TCTGAAGTTT TAAAATATTA CACACTTTGA AATAGCATTT GTACCTTGAA ATACCTGTCT  660
   CTATATATTT TTTAAAACTT CCTTTTTCTT TCATTCCATT TATCATCAAA TAAAGGATGA  720
   ACAGATGTAA CTCAGAAACT GTCAAGCATG CTGAAGAAAG ACCACTGCAG AAAAATTTCT  780
   CCTAGCCTTT TCAAAGGTGT TAGGAAGCAG AAAGGTGATA CAGAATTGGA GAGGTCGGAG  840
   TTTTTGTATT AACTGTATTA AATGCGAATC CCGAGAAAAT TTCCCTTAAC TACGTCCTGT  900
   AGTTATATGG ATATGAAGAC TTATGTGAAC TTTGAAAGAC GTGTCTACAT AAGTTGAAAT  960
   GTCCCCAATG ATTCAGCTGA TGCGCGTTTC TCTACTTGCC CTTTCTAGAG AGGTGCAACG 1020
   GAAGCCAGAA CATTCCTCCT GGAAATTCAA CCTGTTTCGC AGTTTCTCGA GGAATCAGCA 1080
   TTCAGTCAAT CCGGGCCGGG AGCAGTCATC TGTGGTGAGG CTGATTGGCT GGGCAGGAAC 1140
   AGCGCCGGGG CGTGGGCTGA GCACAGCGCT TCGCTCTCTT TGCCACAGGA AGCCTGAGCT 1200
   CATTCGAGTA GCGGCTCTTC CAAGCTCAAA GAAGCAGAGG CCGCTGTTCG TTTCCTTTAG 1260
   GTCTTTCCAC TAAAGTCGGA GTATCTTCTT CCAAGATTTC ACGTCTTGGT GGCCGTTC   1318

### (2) ANGABEN ZU SEQ ID NO: 4:

(i) SEQUENZKENNZEICHEN:
   (A) LÄNGE: 568 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..568
   (D) SONSTIGE ANGABEN:/note= "Sequenz gem. Anspruch 2 d)"
(xi) SEQUENZBESCHREIBUNG: SEQ ID NO: 4:
   CTGAAGAAAG ACCACTGCAG AAAAATTTCT CCTAGCCTTT TCAAAGGTGT TAGGAAGCAG    60
   AAAGGTGATA CAGAATTGGA GAGGTCGGAG TTTTTGTATT AACTGTATTA AATGCGAATC   120
   CCGAGAAAAT TTCCCTTAAC TACGTCCTGT AGTTATATGG ATATGAAGAC TTATGTGAAC   180
   TTTGAAAGAC GTGTCTACAT AAGTTGAAAT GTCCCCAATG ATTCAGCTGA TGCGCGTTTC   240
   TCTACTTGCC CTTTCTAGAG AGGTGCAACG GAAGCCAGAA CATTCCTCCT GGAAATTCAA   300
   CCTGTTTCGC AGTTTCTCGA GGAATCAGCA TTCAGTCAAT CCGGGCCGGG AGCAGTCATC   360
   TGTGGTGAGG CTGATTGGCT GGGCAGGAAC AGCGCCGGGG CGTGGGCTGA GCACAGCGCT   420
   TCGCTCTCTT TGCCACAGGA AGCCTGAGCT CATTCGAGTA GCGGCTCTTC CAAGCTCAAA   480
   GAAGCAGAGG CCGCTGTTCG TTTCCTTTAG GTCTTTCCAC TAAAGTCGGA GTATCTTCTT   540
   CCAAGATTTC ACGTCTTGGT GGCCGTTC                                      568

### (2) ANGABEN ZU SEQ ID NO: 5:

(i) SEQUENZKENNZEICHEN:
   (A) LÄNGE: 351 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..351
   (D) SONSTIGE ANGABEN:/note= "Sequenz gem. Anspruch 3 a)"
(xi) SEQUENZBESCHREIBUNG: SEQ ID NO: 5:
   CTTGCCCTTT CTAGAGAGGT GCAACGGAAG CCAGAACATT CCTCCTGGAA ATTCAACCTG    60
   TTTCGCAGTT TCTCGAGGAA TCAGCATTCA GTCAATCCGG GCCGGGAGCA GTCATCTGTG   120
   GTGAGGCTGA TTGGCTGGGC AGGAACAGCG CCGGGGCGTG GGCTGAGCAC AGCGCTTCGC   180
   TCTCTTTGCC ACAGGAAGCC TGAGCTCATT CGAGTAGCGG CTCTTCCAAG CTCAAAGAAG   240
   CAGAGGCCGC TGTTCGTTTC CTTTAGGTCT TTCCACTAAA GTCGGAGTAT CTTCTTCCAA   300
   GATTTCACGC CTTGGTGGCC GTTCCAAGGA GCGCGAGGTA GGGGCACGCA A            351

### (2) ANGABEN ZU SEQ ID NO: 6:

(i) SEQUENZKENNZEICHEN:
   (A) LÄNGE: 346 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..346
   (D) SONSTIGE ANGABEN:/note= "Sequenz gem. Anspruch 3 b)"
(xi) SEQUENZBESCHREIBUNG: SEQ ID NO: 6:
   CTTGCCCTTT CTAGAGAGGT GCAACGGAAG CCAGAACATT CCTCCTGGAA ATTCAACCTG    60
   TTTCGCAGTT TCTCGAGGAA TCAGCATTCA GTCAATCCGG GCCGGGAGCA GTCATCTGTG   120
   GTGAGGCTGA TTGGCTGGGC AGGAACAGCG CCGGGGCGTG GGCTGAGCAC AGCGCTTCGC   180
   TCTCTTTGCC ACAGGAAGCC TGAGCTCATT CGAGTAGCGG CTCTTCCAAG CTCAAAGAAG   240
   CAGAGGCCGC TGTTCGTTTC CTTTAGGTCT TTCCACTAAA GTCGGAGTAT CTTCTTCCAA   300
   GATTTCACGC CTTGGTGGCC GTTCCAAGGA GCGCGAGGTC ACGCAA                  346

### (2) ANGABEN ZU SEQ ID NO: 7:

(i) SEQUENZKENNZEICHEN:
   (A) LÄNGE: 694 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: -
   (B) LAGE:1..694
   (D) SONSTIGE ANGABEN:/note= "Sequenz gem. Anspruch 4"
(xi) SEQUENZBESCHREIBUNG: SEQ ID NO: 7:
   CCCTTCTAAC CATGGCCAGT GCGTGATGAC TGCTTGGGAG AGGTTATCAT TGGTGTGTCC   60
   CTTTGTCATT AGTAATCACG GGTGACTCCA CGGCAGAAGT CAGGTACACT GGGTCTGTCA  120
   CTCACTCCGT GAGACAGCGG GCAGGTCCCC AAACTTCTCT GATCCTCTCT ATTTCCTGGT  180
   TAGTCAATGG GAGATTAAAC CAAAACCAGA GGAAACATTG TGAGCAAACC AAGAGCACCT  240
   CCTTGTCACC TCAGTTTATG CCGGGCCCTG GCTCCGAGCT ACTGAAACCG CAGCATGGGG  300
   GAGTCTCCTG GGTGGGGTGC ACCATTGACA GGAGGCACTG TTTAAGGCCT CACAAAACGT  360
   GGGCTTCCTC GCCCTGGGCC TGATGGTTCT TGAAGGCCAG GTTCCCTGCA AATCTGGCCT  420
   CTCTTCCCAG TCAAGGCTGA AGTAAAGGGT GAGTTTCCAC TGCTTTTCAC AGTGGGTGTT  480
   CTGGGGGCAA GGAGCTCCCG CGAAGAAGAG GAGGAAAAAC AAGCCTGGTC AGAGGGGCAC  540
   AGACATGTGT CCCTCTGGCC TGGTACGTCG GGACTTCAGC AGCGTCTCAG ATATCACAAT  600
   CGTCCGGGGC AGAAATTGTG ACGTGCCGCT GGGGTAGCTT GGCAGCTGTG GACACCGTTG  660
   CTATGTAAGG CGGGAGGTGA GTCACTGTCT CCAA                              694